# EUROPEAN PATENT APPLICATION

(11) **EP 4 628 579 A1**
(43) Date of publication of application: **08.10.2025**
(21) Application number: 23895684.1
(22) Date of filing: 21.02.2023
(51) Int. Cl.: C12N 9/10, C12N 15/54, C12N 15/63, C12P 13/00, C12P 17/12

(54) **TRANSAMINASE MUTANT AND USE**

(30) Priority: 02.12.2022 CN 202211538995
(71) Applicant: Asymchem Life Science (Tianjin) Co., Ltd., Binhai New Area, Tianjin 300457 (CN)
(72) Inventor: HONG, Hao, Morrisville, NC 27560 (US); JAMES, Gage, Morrisville, NC 27560 (US); ZHANG, Na, Tianjin 300457 (CN); JIAO, Xuecheng, Tianjin 300457 (CN); MA, Yulei, Tianjin 300457 (CN); MU, Huiyan, Tianjin 300457 (CN); MA, Tianjiao, Tianjin 300457 (CN); ZHAO, Tong, Tianjin 300457 (CN)
(74) Representative: Novagraaf Technologies
(86) International application number: PCT/CN2023/077455
(87) International publication number: WO 2024/113508

(57) **Abstract**

Provided are a transaminase mutant and use. The transaminase mutant comprises (a) a protein having an amino acid sequence as set forth in SEQ ID NO: 1; or (b) a protein, which is subjected to amino acid mutation on at least one of the following sites: V315, I91, V124, Y116, A286, C418, T87, S301, S27, V31, R34, M64, A74, R77, S101, T117, N151, L213, T285, T107 or L449, of the amino acid sequence in (a) and has a transaminase activity function; and (c) a protein having 80% or more homology with an amino acid sequence defined in any one of (a) and (b) and having a transaminase function. The problem of poor activity or tolerance of transaminase in industrial production of catalyzing a large-steric-hindrance chiral amine compound in the prior art is solved.

## Description

The present application is based on and claims priority to Chinese Application No. 202211538995.8, filed on December 2, 2022, the disclosure of which is again incorporated into the present application in its entirety.

### Technical Field

The present disclosure relates to the field of enzyme catalysis, specifically to a transaminase mutant and use.

### Background

Chiral amines are structural units of many important bioactive molecules and key intermediates for the synthesis of many chiral drugs. At present, there are three main methods for the synthesis of chiral amines, including chemical methods, biological resolution methods, and biological asymmetric synthesis methods. Among them, chemical methods have disadvantages such as long reaction routes and harsh conditions, the use of toxic transition metal catalysts in synthesis, low product stereoselectivity, and low yield; and the theoretical maximum yield of biological resolution methods is only 50%, and both methods have certain limitations in scale-up production.

The asymmetric synthesis of chiral amines catalyzed by transaminase has attracted increasing attention due to its high selectivity, high conversion rate, mild reaction conditions, and environmental friendliness. It has now become a widely used preparation method for chiral amines. However, most wild-type transaminase substrates have a limited range and are often difficult to synthesize large-steric-hindrance chiral amine compounds (referring to the substituent on the carbonyl side of the catalytic substrate being larger than the methyl group). CN 114875006 A discloses a transaminase mutant that can efficiently synthesize chiral amines, especially large-steric-hindrance chiral amines. However, these mutants have relatively poor stability in industrial applications and are difficult to adapt to extreme environments such as high temperatures and high concentrations of organic solvents in amplification processes.

The method of enzyme evolution can improve the tolerance of transaminase. Cao, J., et al. modified the thermal stability of (R)-type transaminase derived from Aspergillus terreus. Thirteen mutants were constructed by site-directed mutagenesis and three mutants (E133Q, D224K, and E253A) were confirmed to contribute to the improvement of enzyme thermal stability through experiments. Among them, the stability of mutant D224K at 40°C was increased by 4.23 times (Front. Chem. 2021, 9:664156.). In addition, Cai, B., et al. utilized (R) - type transaminase derived from Aspergillus niger Af293 to construct an efficient enzymatic process for producing (R)-α-phenylethylamine. The mutant obtained through directed evolution showed an activity increase of over 3000 times and greatly improved tolerance to isopropylamine (2M). They also achieved a (R)-α-phenylethylamine yield of up to 168 g L⁻¹ d⁻¹ on an industrial trial scale (Org. Process Res. Dev. 2022, 26, 7, 2004-2012).

Therefore, in order to further adapt to the industrial production requirements of synthesizing large-steric-hindrance chiral amines, we can improve the stability of transaminases under extreme conditions through enzyme evolution. To further promote the immobilization and continuous application of transaminase, improve production efficiency, reduce industrial production costs, and decrease the emission of three industrial wastes.

### Summary

The main objective of the present disclosure is to provide a transaminase mutant and use to solve the problem of poor activity or tolerance of transaminase in industrial production of catalyzing a large-steric-hindrance chiral amine compound in the prior art.

In order to achieve the above-mentioned objectives, according to the first aspect of the present disclosure, a transaminase mutant is provided, comprising (a) a protein having an amino acid sequence as set forth in SEQ ID NO: 1; or (b) a protein, which is subjected to amino acid mutation on at least one of the following sites: V315, I91, V124, Y116, A286, C418, T87, S301, S27, V31, R34, M64, A74, R77, S101, T117, N151, L213, T285, T107 or L449, of the amino acid sequence in (a) and has a transaminase activity function; and (c) a protein having 80% or more homology with an amino acid sequence defined in any one of (a) and (b) and having a transaminase function.

Furthermore, the amino acid mutation of (b) is each independently selected from the following: V315T, V315R, V315D, V315A, V315K or V315H; I91Q, I91M, I91N, I91G, I91F or I91D; V124C, V124S, V124Y, V124P, V124M, V124A or V124I; Y116F, Y116Y, Y116A, Y116R, Y116S, Y116G, Y116H or Y116L; A286I, A286R, A286Q, A286F, A286D, A286M or A286I; C418L, C418W, C418R, C418D, C418F or C418A; T87A, T87Y, T87N, T87V, T87F or T87E; S301A, S301G, S301H, S301K, S301W, S301I or S301N; S27A, S27V, S27N, S27M, S27E, S27R or S27; S101A, S101R, S101Y or S101H; R34H, R34K, R34N, R34T, R34F or R34M; T117V, T117M, T117L, T117T, T117R or T117S; T285M, T285Y, T285I, T285A or T285G; T107S, T107Q, T107A or T107G; wherein the letter before a number represents the original amino acid, and a letter after the number represents a mutated amino acid; preferably, the protein in the (c) is a protein having more than 85%, preferably more than 90%, more preferably more than 95%, and further more preferably more than 99% identity with the amino acid sequence defined in any of (a) and (b) and having the function of transaminase.

Furthermore, the transaminase mutant comprises any one of the following amino acid mutations: V315T; V315R; V315D; V315A; V315K; V315H; V315T+I91Q; V315T+I91M; V315T+I91N; V315T+I91G; V315T+I91F; V315T+I91D; V315T+V124C; V315T+V124S; V315T+V124Y; V315T+V124P; V315T+V124M; V315T+V124A; V315T+V124C+I91N; V315T+V124C+I91Q; V315T+V124C+I91M; V315T+V124C+I91G; V315T+V124C+I91F; V315T+I91Q+V124S; V315T+I91Q+V124I; V315T+I91Q+V124P; V315T+I91Q+V124M; V315T+I91Q+V124A; V315T+I91Q+V124Y; V315T+I91Q+V124S+Y116F; V315T+I91Q+V124S+Y116Y; V315T+I91Q+V124S+Y116A; V315T+I91Q+V124S+Y116R; V315T+V124C+I91N+Y116F; V315T+V124C+I91N+Y116A; V315T+V124C+I91N+Y116S; V315T+V124C+I91N+Y116G; V315T+V124C+I91N+Y116H; V315T+V124C+I91N+Y116L; V315T+V124C+I91N+Y116F+A286I; V315T+V124C+I91N+Y116F+A286R; V315T+V124C+I91N+Y116F+A286Q; V315T+V124C+I91N+Y116F+A286F; V315T+V124C+I91N+Y116F+A286D; V315T+V124C+I91N+Y116F+A286M; V315T+V124C+I91N+Y116F+A286I+C418L; V315T+V124C+I91N+Y116F+A286I+C418W; V315T+V124C+I91N+Y116F+A286I+C418R; V315T+V124C+I91N+Y116F+A286I+C418D; V315T+V124C+I91N+Y116F+A286I+C418F; V315T+V124C+I91N+Y116F+A286I+C418A; V315T+V124C+I91N+Y116F+A286I+T87A; V315T+V124C+I91N+Y116F+A286I+T87Y; V315T+V124C+I91N+Y116F+A286I+T87N; V315T+V124C+I91N+Y116F+A286I+T87V; V315T+V124C+I91N+Y116F+A286I+T87F; V315T+V124C+I91N+Y116F+A286I+T87E; V315T+V124C+I91N+Y116F+A286I+T87A+S301A; V315T+V124C+I91N+Y116F+A286I+T87A+S301G; V315T+V124C+I91N+Y116F+A286I+T87A+S301H; V315T+V124C+I91N+Y116F+A286I+T87A+S301K; V315T+V124C+I91N+Y116F+A286I+T87A+S301W; V315T+V124C+I91N+Y116F+A286I+T87A+S27A; V315T+V124C+I91N+Y116F+A286I+T87A+S27V; V315T+V124C+I91N+Y116F+A286I+T87A+S27N; V315T+V124C+I91N+Y116F+A286I+T87A+S27M; V315T+V124C+I91N+Y116F+A286I+T87A+S27E; V315T+V124C+I91N+Y116F+A286I+T87A+S27R; V315T+V124C+I91N+Y116F+A286I+T87A+S27A+S101A; V315T+V124C+I91N+Y116F+A286I+T87A+S27A+S101R; V315T+V124C+I91N+Y116F+A286I+T87A+S27A+S101Y; V315T+V124C+I91N+Y116F+A286I+T87A+S27A+S101H; V315T+V124C+I91N+Y116F+A286I+T87A+S27A+R34H; V315T+V124C+I91N+Y116F+A286I+T87A+S27A+R34K; V315T+V124C+I91N+Y116F+A286I+T87A+S27A+R34N; V315T+V124C+I91N+Y116F+A286I+T87A+S27A+R34T; V315T+V124C+I91N+Y116F+A286I+T87A+S27A+R34F; V315T+V124C+I91N+Y116F+A286I+T87A+S27A+T117V; V315T+V124C+I91N+Y116F+A286I+T87A+S27A+T117M; V315T+V124C+I91N+Y116F+A286I+T87A+S27A+S301K; V315T+V124C+I91N+Y116F+A286I+T87A+S27A+S301I; V315T+V124C+I91N+Y116F+A286I+T87A+S27A+S301G; V315T+V124C+I91N+Y116F+A286I+T87A+S27A+S301W; V315T+V124C+I91N+Y116F+A286I+T87A+S27A+S101A+R34H; V315T+V124C+I91N+Y116F+A286I+T87A+S27A+S101A+R34T; V315T+V124C+I91N+Y116F+A286I+T87A+S27A+S101A+R34N; V315T+V124C+I91N+Y116F+A286I+T87A+S27A+S101A+R34F; V315T+V124C+I91N+Y116F+A286I+T87A+S27A+S101A+R34M; V315T+V124C+I91N+Y116F+A286I+T87A+S27A+S101A+S301M; V315T+V124C+I91N+Y116F+A286I+T87A+S27A+S101A+S301K; V315T+V124C+I91N+Y116F+A286I+T87A+S27A+S101A+S301I; V315T+V124C+I91N+Y116F+A286I+T87A+S27A+S101A+S301N; V315T+V124C+I91N+Y116F+A286I+T87A+S27A+S101A+S301A; V315T+V124C+I91N+Y116F+A286I+T87A+S27A+S101A+R34H+T117V; V315T+V124C+I91N+Y116F+A286I+T87A+S27A+S101A+R34H+T117L; V315T+V124C+I91N+Y116F+A286I+T87A+S27A+S101A+R34H+T117T; V315T+V124C+I91N+Y116F+A286I+T87A+S27A+S101A+R34H+T117R; V315T+V124C+I91N+Y116F+A286I+T87A+S27A+S101A+R34H+T117S; V315T+V124C+I91N+Y116F+A286I+T87A+S27A+S101A+R34H+T117V+T285M; V315T+V124C+I91N+Y116F+A286I+T87A+S27A+S101A+R34H+T117V+T285Y; V315T+V124C+I91N+Y116F+A286I+T87A+S27A+S101A+R34H+T117V+T285I; V315T+V124C+I91N+Y116F+A286I+T87A+S27A+S101A+R34H+T117V+T285A; V315T+V124C+I91N+Y116F+A286I+T87A+S27A+S101A+R34H+T117V+T285G; V315T+V124C+I91N+Y116F+A286I+T87A+S27A+S101A+R34H+T117V+T107S; V315T+V124C+I91N+Y116F+A286I+T87A+S27A+S101A+R34H+T117V+T107Q; V315T+V124C+I91N+Y116F+A286I+T87A+S27A+S101A+R34H+T117V+T107A; and V315T+V124C+I91N+Y116F+A286I+T87A+S27A+S101A+R34H+T117V+T107G.

In order to achieve the above-mentioned objectives, according to the second aspect of the present disclosure, a DNA molecule encoding the above-mentioned transaminase mutant is provided.

In order to achieve the above-mentioned objectives, according to the third aspect of the present disclosure, a recombinant plasmid connected with the above-mentioned DNA molecule is provided.

In order to achieve the above-mentioned objectives, according to the fourth aspect of the present disclosure, a host cell transformed with the above-mentioned recombinant plasmid is provided.

In order to achieve the above-mentioned objectives, according to the fifth aspect of the present disclosure, a preparation method for a chiral amine compound is provided, comprising performing a transamination reaction with the above-mentioned transaminase mutant and a ketone-based substrate as shown in a formula I under the action of an amino donor, to obtain the chiral amine compound,

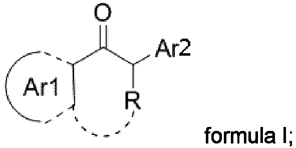

Ar1 is selected from a first substituted aryl group, a first unsubstituted aryl group, a substituted arylene group or an unsubstituted arylene group, a substituted heteroarylene group or an unsubstituted heteroarylene group; Ar2 is selected from a second substituted aryl group, a second unsubstituted aryl group, a substituted cycloalkyl group or an unsubstituted cycloalkyl group, an alkyl group or an alkylene group; R is selected from H, an alkyl group, an alkylene group or an alkylidene group, the number of C atoms in the alkyl group, alkylene group or alkylidene group is selected from 1 to 5, and the alkyl group, alkylene group or alkylidene group includes a substituted alkyl group, alkylene group or alkylidene group, or an unsubstituted alkyl group, alkylene group or alkylidene group; when R is selected from an alkyl group, alkylene group or alkylidene group, the alkyl group, alkylene group or alkylidene group is linked to the Ar1 and/or the Ar2 to form a ring; the substituents in the first substituted aryl group, the substituted arylene group, the substituted heteroarylene group, the second substituted aryl group, or the substituted alkyl group, alkylene group or alkylidyne group are each independently selected from a halogen, a hydroxyl group, an amino group, a methyl group, an ethyl group or -CH₂CH₂OH; and the heteroatoms in the substituted heteroarylene group are selected from N, O or S.

Furthermore, the substituents in the first substituted aryl group, the second substituted aryl group or the substituted arylene group are each independently selected from the halogen.

Furthermore, the substituent groups are each independently located at any one or more positions of ortho-, meta- or para-position of the first substituted aryl group, the second substituted aryl group or the substituted arylene group.

Furthermore, the halogen is selected from F, Cl or Br.

Furthermore, the Ar1 is selected from the unsubstituted heteroarylene group, the Ar2 is selected from the second substituted aryl group, and the substituent of the second substituted aryl group is selected from the halogen, the R is selected from the substituted alkylene group, the number of C atoms in the substituted alkylene group is selected from 1 to 5, and the substituent is selected from a hydroxyl group, and the substituted alkylene group is linked to the Ar1 to form a ring.

Furthermore, the Ar1 is selected from the first substituted aryl group or the first unsubstituted aryl group, and the substituent of the first substituted aryl group is selected from a methyl group or Cl, the Ar2 is selected from the unsubstituted cycloalkyl group, alkyl group or second unsubstituted aryl group, the number of C atoms in the unsubstituted cycloalkyl group is selected from 3 to 5, and the alkyl group is selected from an isopropyl group or an ethyl group, the R is selected from the alkyl group, and the alkyl group is selected from an isopropyl group, a methyl group, or an ethyl group.

Furthermore, the Ar1 is selected from the first substituted aryl group or the first unsubstituted aryl group, and the substituent of the first substituted aryl group is selected from a methyl group or - CH₂CH₂OH, the Ar2 is selected from the second unsubstituted aryl group or alkyl group, the alkyl group is selected from a methyl group or an isopropyl group, the R is selected from a substituted alkylene group, the number of C atoms in the substituted alkylene group is selected from 1 to 5, and the substituent is selected from a hydroxyl group, and the substituted alkylene group is linked to the Ar1 to form a ring.

Furthermore, the ketone-based substrate is selected from 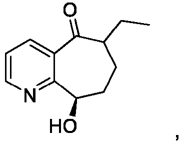 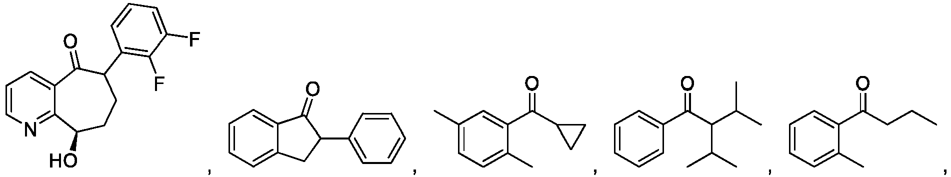 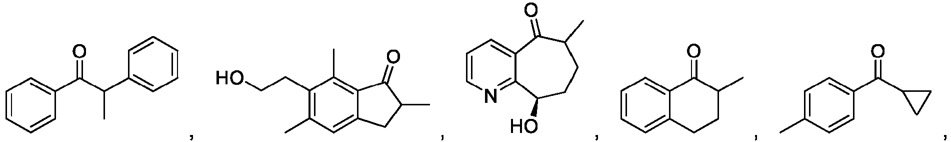 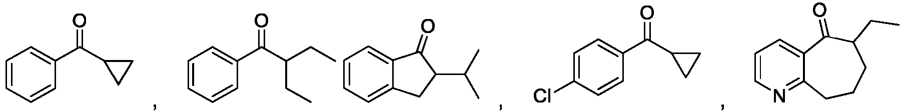 or

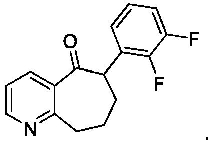

By applying the technical solution of the present disclosure, a transaminase mutant (SEQ ID NO: 1) derived from *Chromobacterium violaceum* was used as the parent, and protein engineering modifications such as saturation mutation and combinatorial mutation were carried out to obtain transaminase mutants with greatly improved activity and tolerance in catalyzing the reaction of a large-steric-hindrance chiral amine.

### Detailed Description of the Embodiments

It should be noted that the embodiments and the features of the embodiments in the present application can be combined with each other under the circumstances that there is no conflict. The present disclosure will be described in detail below with reference to embodiments.

### Terminology explanation:

Large-steric-hindrance chiral compounds: in the present application, it refers to ketone compounds where the group next to the prochiral carbonyl group is larger than the methyl group. Among them, the group larger than the methyl group can be ethyl, propyl, tert butyl, or phenyl tert butyl.

As mentioned in the background technology, there are various shortcomings in the chemical synthesis and biological resolution methods of chiral amines. The synthesis of chiral amines using biological asymmetric methods can have higher efficiency and milder reaction conditions. However, due to the limited substrate range of most wild-type transaminases, transaminases often suffer from low activity and poor tolerance when synthesizing large-steric-hindrance chiral amine compounds, making it impossible to produce them through scale-up processes.

Therefore, in the present application, the inventors attempt to modify transaminase through enzyme evolution to improve its various properties under extreme conditions, enabling it to increase production efficiency in industrial production. Therefore, a series of protection schemes are proposed in the present application.

In the first typical embodiment of the present application, a transaminase mutant is provided, comprising: (a) a protein having an amino acid sequence as set forth in SEQ ID NO: 1; or (b) a protein, which is subjected to amino acid mutation on at least one of the following sites: V315, I91, V124, Y116, A286, C418, T87, S301, S27, V31, R34, M64, A74, R77, S101, T117, N151, L213, T285, T107 or L449, of the amino acid sequence in (a) and has a transaminase activity function; and (c) a protein having 80% or more homology with an amino acid sequence defined in any one of (a) and (b) and having a transaminase function.

The above-mentioned amino acid sequence as set forth in SEQ ID NO: 1 is a transaminase mutant derived from *Chromobacterium violaceum.* Through computer simulation analysis of homologous modeling of the amino acid sequence and molecular docking with different large-steric-hindrance ketone compounds, 19 amino acid residues were identified, comprising S27, V31, R34, M64, A74, R77, T87, I91, S101, Y116, T117, V124, N151, L213, T285, A286, V315, C418, and L449. These amino acid sites may affect protein catalytic activity and stability. By mutating the above-mentioned amino acid sites, proteins with transaminase function and even enhanced transaminase function can be obtained. For the protein obtained above, changes can be made at non-critical mutation sites and non-active sites, resulting in a protein with over 80% identity to the above-mentioned amino acid sequence and transaminase function.

The sequence of SEQ ID NO: 1 is as follows:

MQKQRTCSQWRELDAAHHLHPFTDTASLNQVGARVMTRGEGVYLWDCEGNKIIDGMAGAW
CVNMGYGRKDFAEAARRQMEELSFMHTADGITHPAVVELSSLLAEVTPAGFDRVFYTNSGSESVD
CMIRMVRRYWDVQGKPEKKTLIGRWNGYSGSTIGGASLSGFKEMHEQGDLPIPGVAHIEQPWWY
KHGKDMTPDEFGVVAARWLEEKILEIGADKVAAFVGEPIQGAGGAIVPPATYWPEIERICRKYDVLL
VADEVICGFGRTGEWFGHQHFGFQPDLFTAAKGLSSGYQPIGAVSVGKRVAEGLIAGGVFYHGHT
TSGHPVCAAVAHANVAALRDEGIVQRVKDDIGPYMQKRWRETLSRFEHVDDVRGVGMLAAFTLVK
NKAKRELFPDFGEIGSLCRDIFVRNNLIMDICGDHIVAAPPLVMTRAEVDEMLAVAERCLEELEQSL
KARGLA.

In a preferred embodiment, the amino acid mutation of (b) is each independently selected from the following: V315T, V315R, V315D, V315A, V315K or V315H; I91Q, I91M, I91N, I91G, I91F or I91D; V124C, V124S, V124Y, V124P, V124M, V124A or V124I; Y116F, Y116Y, Y116A, Y116R, Y116S, Y116G, Y116H or Y116L; A286I, A286R, A286Q, A286F, A286D, A286M or A286I; C418L, C418W, C418R, C418D, C418F or C418A; T87A, T87Y, T87N, T87V, T87F or T87E; S301A, S301G, S301H, S301K, S301W, S301I, S301M,or S301N; S27A, S27V, S27N, S27M, S27E, S27R or S27; S101A, S101R, S101Y or S101H; R34H, R34K, R34N, R34T, R34F or R34M; T117V, T117M, T117L, T117T, T117R or T117S; T285M, T285Y, T285I, T285A or T285G; T107S, T107Q, T107A or T107G; wherein the letter before a number represents the original amino acid, and a letter after the number represents a mutated amino acid; preferably, the protein in the (c) is a protein having more than 85%, preferably more than 90%, more preferably more than 95%, and further more preferably more than 99% identity with the amino acid sequence defined in any of (a) and (b) and having the function of transaminase.

As used herein, the abbreviations for amino acid residues are as follows: alanine (Ala; A), asparagine (Asn; N), aspartic acid (Asp; D), arginine (Arg; R), cysteine (Cys; C), glutamic acid (Glu; E), glutamine (Gln; Q), glycine (Gly; G), histidine (His; H), isoleucine (lle; I), leucine (Leu; L), lysine (Lys; K), methionine (Met; M), phenylalanine (Phe; F), proline (Pro; P), serine (Ser; S), threonine. (Thr; T), tryptophan (Trp; W), tyrosine (Tyr; Y), and valine (Val; V).

The rules of replacement, substitution, etc. generally determine which amino acids have similar properties and the effect after substitution is also similar. For example, conservative amino acid substitutions can occur in the above-mentioned homologous proteins. "Conservative amino acid substitutions" include but are not limited to:
hydrophobic amino acids (Ala, Cys, Gly, Pro, Met, Val, Ile, Leu) are replaced by other hydrophobic amino acids;
hydrophobic amino acids with thick side chains (Phe, Tyr, Trp) are replaced by other hydrophobic amino acids with thick side chains;
amino acids with positively charged side chains (Arg, His, Lys) are replaced by other amino acids with positively charged side chains;
amino acids with polar uncharged side chains (Ser, Thr, Asn, Gln) are replaced by other amino acids with polar uncharged side chains.

Those skilled in the art can also perform conservative substitution of amino acids based on amino acid substitution rules such as the "blosum62 scoring matrix" well known to those skilled in the art in the prior art.

In the present application, the applicant continued to explore the above-mentioned active sites and found that mutations in the active sites to different amino acids resulted in differences in protein activity, and specific mutations could enhance transaminase activity. Through experimental exploration, it was found that the above-mentioned specific mutations at the active site could result in proteins with enhanced activity. For the amino acid mutation sites of transaminase proteins, flexible selection and combination can be made among the above-mentioned mutations.

In a preferred embodiment, the mutation of the transaminase mutant comprises any one of the following amino acid mutations:
V315T; V315R; V315D; V315A; V315K; V315H; V315T+I91Q; V315T+I91M; V315T+I91N; V315T+I91G; V315T+I91F; V315T+I91D; V315T+V124C; V315T+V124S; V315T+V124Y; V315T+V124P; V315T+V124M; V315T+V124A; V315T+V124C+I91N; V315T+V124C+I91Q; V315T+V124C+I91M; V315T+V124C+I91G; V315T+V124C+I91F; V315T+I91Q+V124S; V315T+I91Q+V124I; V315T+I91Q+V124P; V315T+I91Q+V124M; V315T+I91Q+V124A; V315T+I91Q+V124Y; V315T+I91Q+V124S+Y116F; V315T+I91Q+V124S+Y116Y; V315T+I91Q+V124S+Y116A; V315T+I91Q+V124S+Y116R; V315T+V124C+I91N+Y116F; V315T+V124C+I91N+Y116A; V315T+V124C+I91N+Y116S; V315T+V124C+I91N+Y116G; V315T+V124C+I91N+Y116H; V315T+V124C+I91N+Y116L; V315T+V124C+I91N+Y116F+A286I; V315T+V124C+I91N+Y116F+A286R; V315T+V124C+I91N+Y116F+A286Q; V315T+V124C+I91N+Y116F+A286F; V315T+V124C+I91N+Y116F+A286D; V315T+V124C+I91N+Y116F+A286M; V315T+V124C+I91N+Y116F+A286I+C418L; V315T+V124C+I91N+Y116F+A286I+C418W; V315T+V124C+I91N+Y116F+A286I+C418R; V315T+V124C+I91N+Y116F+A286I+C418D; V315T+V124C+I91N+Y116F+A286I+C418F; V315T+V124C+I91N+Y116F+A286I+C418A; V315T+V124C+I91N+Y116F+A286I+T87A; V315T+V124C+I91N+Y116F+A286I+T87Y; V315T+V124C+I91N+Y116F+A286I+T87N; V315T+V124C+I91N+Y116F+A286I+T87V; V315T+V124C+I91N+Y116F+A286I+T87F; V315T+V124C+I91N+Y116F+A286I+T87E; V315T+V124C+I91N+Y116F+A286I+T87A+S301A; V315T+V124C+I91N+Y116F+A286I+T87A+S301G; V315T+V124C+I91N+Y116F+A286I+T87A+S301H; V315T+V124C+I91N+Y116F+A286I+T87A+S301K; V315T+V124C+I91N+Y116F+A286I+T87A+S301W; V315T+V124C+I91N+Y116F+A286I+T87A+S27A; V315T+V124C+I91N+Y116F+A286I+T87A+S27V; V315T+V124C+I91N+Y116F+A286I+T87A+S27N; V315T+V124C+I91N+Y116F+A286I+T87A+S27M; V315T+V124C+I91N+Y116F+A286I+T87A+S27E; V315T+V124C+I91N+Y116F+A286I+T87A+S27R; V315T+V124C+I91N+Y116F+A286I+T87A+S27A+S101A; V315T+V124C+I91N+Y116F+A286I+T87A+S27A+S101R; V315T+V124C+I91N+Y116F+A286I+T87A+S27A+S101Y; V315T+V124C+I91N+Y116F+A286I+T87A+S27A+S101H; V315T+V124C+I91N+Y116F+A286I+T87A+S27A+R34H; V315T+V124C+I91N+Y116F+A286I+T87A+S27A+R34K; V315T+V124C+I91N+Y116F+A286I+T87A+S27A+R34N; V315T+V124C+I91N+Y116F+A286I+T87A+S27A+R34T; V315T+V124C+I91N+Y116F+A286I+T87A+S27A+R34F; V315T+V124C+I91N+Y116F+A286I+T87A+S27A+T117V; V315T+V124C+I91N+Y116F+A286I+T87A+S27A+T117M; V315T+V124C+I91N+Y116F+A286I+T87A+S27A+S301K; V315T+V124C+I91N+Y116F+A286I+T87A+S27A+S301I; V315T+V124C+I91N+Y116F+A286I+T87A+S27A+S301G; V315T+V124C+I91N+Y116F+A286I+T87A+S27A+S301W; V315T+V124C+I91N+Y116F+A286I+T87A+S27A+S101A+R34H; V315T+V124C+I91N+Y116F+A286I+T87A+S27A+S101A+R34T; V315T+V124C+I91N+Y116F+A286I+T87A+S27A+S101A+R34N; V315T+V124C+I91N+Y116F+A286I+T87A+S27A+S101A+R34F; V315T+V124C+I91N+Y116F+A286I+T87A+S27A+S101A+R34M; V315T+V124C+I91N+Y116F+A286I+T87A+S27A+S101A+S301M; V315T+V124C+I91N+Y116F+A286I+T87A+S27A+S101A+S301K; V315T+V124C+I91N+Y116F+A286I+T87A+S27A+S101A+S301I; V315T+V124C+I91N+Y116F+A286I+T87A+S27A+S101A+S301N; V315T+V124C+I91N+Y116F+A286I+T87A+S27A+S101A+S301A; V315T+V124C+I91N+Y116F+A286I+T87A+S27A+S101A+R34H+T117V; V315T+V124C+I91N+Y116F+A286I+T87A+S27A+S101A+R34H+T117L; V315T+V124C+I91N+Y116F+A286I+T87A+S27A+S101A+R34H+T117T; V315T+V124C+I91N+Y116F+A286I+T87A+S27A+S101A+R34H+T117R; V315T+V124C+I91N+Y116F+A286I+T87A+S27A+S101A+R34H+T117S; V315T+V124C+I91N+Y116F+A286I+T87A+S27A+S101A+R34H+T117V+T285M; V315T+V124C+I91N+Y116F+A286I+T87A+S27A+S101A+R34H+T117V+T285Y; V315T+V124C+I91N+Y116F+A286I+T87A+S27A+S101A+R34H+T117V+T285I; V315T+V124C+I91N+Y116F+A286I+T87A+S27A+S101A+R34H+T117V+T285A; V315T+V124C+I91N+Y116F+A286I+T87A+S27A+S101A+R34H+T117V+T285G; V315T+V124C+I91N+Y116F+A286I+T87A+S27A+S101A+R34H+T117V+T107S; V315T+V124C+I91N+Y116F+A286I+T87A+S27A+S101A+R34H+T117V+T107Q; V315T+V124C+I91N+Y116F+A286I+T87A+S27A+S101A+R34H+T117V+T107A; V315T+V124C+I91N+Y116F+A286I+T87A+S27A+S101A+R34H+T117V+T107G.

The above-mentioned amino acid mutations were all experimentally explored in the embodiments of the present application, and all have transaminase activity. Compared with the parent having an amino acid sequence as set forth in SEQ ID NO: 1, transaminase mutants with higher stability, high tolerance to extreme environments, and high enzyme activity could be obtained, which could be used for industrial scale-up production.

In the second typical embodiment of the present application, a DNA molecule encoding the above-mentioned transaminase mutant is provided.

In the third typical embodiment of the present application, a recombinant plasmid connected with the above-mentioned DNA molecule is provided.

The above-mentioned DNA can encode the above-mentioned transaminase mutant and can be connected to a recombinant plasmid to form a circular DNA. The above-mentioned DNA and recombinant plasmid can be transcribed and translated under the action of RNA polymerase, ribosome, tRNA, etc., thereby obtaining the above-mentioned transaminase mutant.

In the fourth typical embodiment of the present application, a host cell transformed with the above-mentioned recombinant plasmid is provided. The host cell can be a prokaryotic cell or a eukaryotic cell. Specifically, the prokaryotic cell can be Escherichia coli, and the eukaryotic cell can be yeast.

By utilizing the above-mentioned host cell, it is possible to replicate a recombinant plasmid in the host cell and transcribe and translate the DNA molecule carried on the recombinant plasmid, resulting in a large number of transaminase mutants. By utilizing the prior art, the host cell can be performed protein purification after disruption, catalysis by crude enzymes after disruption, or other methods to obtain a transaminase mutant, which can then be used for subsequent catalysis of amine compounds. The host cell is a non-plant derived host cell.

In the fifth typical embodiment of the present application, a method for preparing a chiral amine compound is provided, comprising performing a transamination reaction with the above-mentioned transaminase mutant and a ketone-based substrate as shown in a formula I under the action of an amino donor, to obtain the chiral amine compound; Ar1 is selected from a first substituted aryl group, a first unsubstituted aryl group, a substituted arylene group or an unsubstituted arylene group, a substituted heteroarylene group or an unsubstituted heteroarylene group; Ar2 is selected from a second substituted aryl group, a second unsubstituted aryl group, a substituted cycloalkyl group or an unsubstituted cycloalkyl group, an alkyl group or an alkylene group; R is selected from H, an alkyl group, an alkylene group or an alkylidene group, the number of C atoms in the alkyl group, alkylene group or alkylidene group is selected from 1 to 5, and the alkyl group, alkylene group or alkylidene group includes a substituted alkyl group, alkylene group or alkylidene group, or an unsubstituted alkyl group, alkylene group or alkylidene group; when R is selected from an alkyl group, alkylene group or alkylidene group, the alkyl group, alkylene group or alkylidene group is linked to the Ar1 and/or the Ar2 to form a ring; the substituents in the first substituted aryl group, the substituted arylene group, the substituted heteroarylene group, the second substituted aryl group, or the substituted alkyl group, alkylene group or alkylidyne group are each independently selected from a halogen, a hydroxyl group, an amino group, a methyl group, an ethyl group or -CH₂CH₂OH; and the heteroatoms in the substituted heteroarylene group are selected from N, O or S.

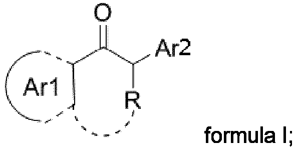

Using the above-mentioned preparation method, the above-mentioned transaminase mutant is used to perform a transamination reaction on a ketone compound as shown in a formula I under the action of an amino donor, obtaining the chiral amine compound. The above-mentioned transaminase mutant can perform chiral catalysis on the above-mentioned ketone compound and synthesize the required large-steric-hindrance chiral amine. The activity and tolerance of transaminase is significantly increased, enabling it to catalyze under industrial production conditions such as extreme environments, improve production efficiency, and reduce industrial production costs.

In a preferred embodiment, the substituents in the first substituted aryl group, the second substituted aryl group or the substituted arylene group are each independently selected from the halogen.

In a preferred embodiment, the substituent groups are each independently located at any one or more positions of ortho-, meta- or para-position of the first substituted aryl group, the second substituted aryl group or the substituted arylene group.

In a preferred embodiment, the halogen is selected from F, Cl or Br.

In a preferred embodiment, the Ar1 is selected from the unsubstituted heteroarylene group, the Ar2 is selected from the second substituted aryl group, and the substituent of the second substituted aryl group is selected from the halogen, the R is selected from the substituted alkylene group, the number of C atoms in the substituted alkylene group is selected from 1 to 5, and the substituent is selected from a hydroxyl group, and the substituted alkylene group is linked to the Ar1 to form a ring.

In a preferred embodiment, the Ar1 is selected from the first substituted aryl group or the first unsubstituted aryl group, and the substituent of the first substituted aryl group is selected from a methyl group or Cl, the Ar2 is selected from the unsubstituted cycloalkyl group, alkyl group or second unsubstituted aryl group, the number of C atoms in the unsubstituted cycloalkyl group is selected from 3 to 5, and the alkyl group is selected from an isopropyl group or an ethyl group, the R is selected from the alkyl group, and the alkyl group is selected from an isopropyl group, a methyl group, or an ethyl group.

In a preferred embodiment, the Ar1 is selected from the first substituted aryl group or the first unsubstituted aryl group, and the substituent of the first substituted aryl group is selected from a methyl group or -CH₂CH₂OH, the Ar2 is selected from the second unsubstituted aryl group or alkyl group, the alkyl group is selected from a methyl group or an isopropyl group, the R is selected from a substituted alkylene group, the number of C atoms in the substituted alkylene group is selected from 1 to 5, and the substituent is selected from a hydroxyl group, and the substituted alkylene group is linked to the Ar1 to form a ring.

In a preferred embodiment, the ketone compound is selected from the group consisting of

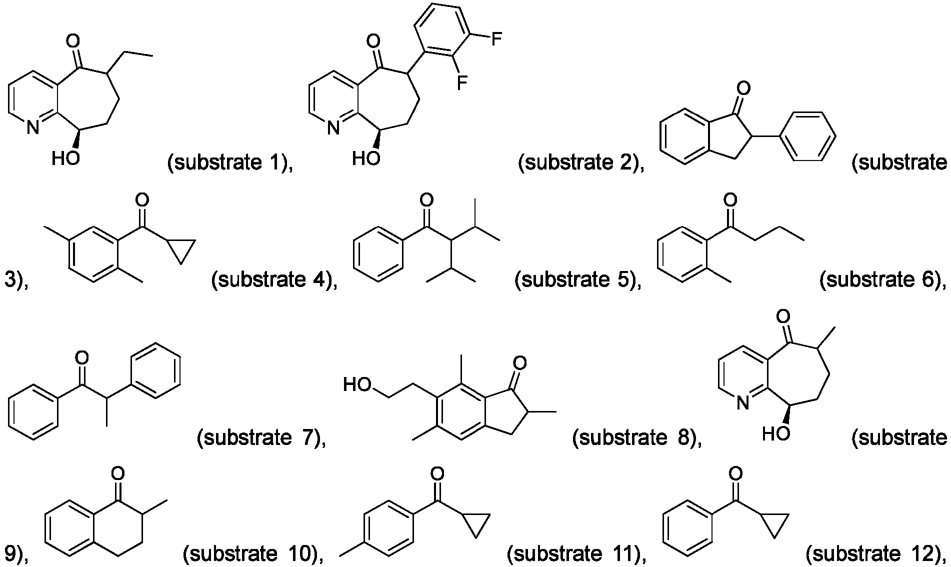

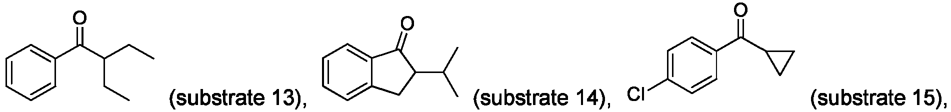

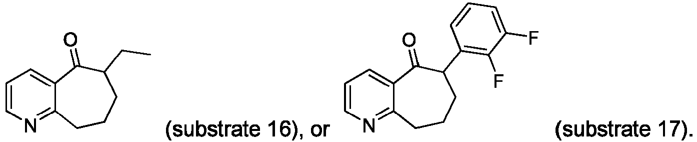

In the present application, the inventors performed homology modeling on the selected mutants based on the three-dimensional structure of the protein (PDB: 4BA5) that had already been resolved. Different large-steric-hindrance ketone compounds were docked based on the model structure, and under the guidance of the strategy to improve protein stability, 19 residues that might affect protein catalytic activity and stability were selected for modification. These residues include: S27, V31, R34, M64, A74, R77, T87, I91, S101, Y116, T117, V124, N151, L213, T285, A286, V315, C418, and L449. The ways of protein modification include saturation mutation and combinatorial mutation.

Saturation mutation is a method of obtaining a mutant with amino acids at target sites replaced by 19 other amino acids in a short period of time by modifying the encoding genes of the target protein. This method is not only a powerful tool for protein directed modification, but also an important means for studying the relationship between protein structure and function. Saturation mutation often results in a more ideal evolution system than single point mutation. For the problems that site-directed mutagenesis methods cannot solve, it is precisely the unique advantage of saturation mutation methods. The construction of a saturation mutation was carried out using whole plasmid PCR, followed by digestion of the PCR product with DPNI enzyme to remove the template and transformation into *Escherichia coli* BL21 (DE3). The screening of mutants often requires high-throughput screening methods, therefore, we have developed the following methods for screening mutant tolerance.

The following high-throughput screening methods were developed to screen mutant libraries:
1. mutant culture: 300 µL of LB medium was added to each well of the 96 well plate, the single clone on the agar plate was inoculated into the deep well 96 well plate, and cultured overnight at 37°C and 200 rpm; Qpix was used to transfer overnight cultured bacterial solution to another 96 well plate each well of which was added with 800 µL of LB medium, cultured at 37°C and 200 rpm for 5 hours until the OD600 of the bacterial solution in the 96 well plate reached 0.6-0.9. Then, Qpix was used to add IPTG solution to the 96 well plate again, so that the final concentration of IPTG in the plate is 0.1 mM. The solution was induced overnight at 25°C and 200 rpm for about 16 hours, and centrifuged at 4000 rpm for 5 minutes. The supernatant was discarded, and whole cells were used to react.
2. 96 well plate high-throughput screening system:
   8 µL of PLP mother liquor (1 mg/mL), 3.5 µL of 6 M isopropylamine hydrochloride (20 eq), and 0.1 M Tris-Cl 9.0 were added to 100 µL and mixed well, and the mixture was divided into 96 well plates with bacterial sludge in each well. Finally, the substrate mother liquor (0.3 mg of substrate dissolved in different concentrations of methanol) was added, mixed well, shaken at a constant temperature of 50°C and 700 rpm, and reacted for 18 hours. Afterwards, 3 times the volume of methanol was added to each well, centrifuged, and the supernatant sample was sent to UPLC for analysis.

Through the initial screening of mutants mentioned above, mutants with improved characters were obtained. Then, the optimal mutant would be induced and cultured in a 2 L shake flask (optimal conditions for induction expression: 25°C, 0.2 mM IPTG overnight induction), centrifuged to obtain bacterial sludge, and crude enzyme solution would be obtained by ultrasonic disruption of cells. Finally, g-level reactions would be performed to confirm the characters of the optimal mutant. Saturation mutations at a single site often did not have a significant improvement effect, so multiple rounds of iterative saturation mutations were generally required to obtain mutants with significantly improved characters.

On the basis of obtaining mutants with increased activity through saturation mutation, beneficial amino acid sites screened could be combined to obtain mutants with better characters. The construction method for double point mutations in combination mutations was the same as that for single point mutations, using whole plasmid PCR. Multiple point mutations that simultaneously mutated two or more sites were amplified by overlapping extension PCR to obtain mutated genes containing multiple point mutations. After restriction endonuclease digestion at both ends, the genes were ligated to an expression vector, transformed into *Escherichia coli* cells, then coated in LB culture dishes containing 100 µg/mL ampicillin and incubated overnight at 37°C to obtain a combination mutant. After the above-mentioned combination mutant was identified by sequencing, it was induced and cultured in 2 L shake flasks as described above for reaction validation.

After multiple rounds of evolution, a series of transaminase mutants were obtained, and it was found that the activity and tolerance of these mutants was greatly improved in catalyzing the reaction of large-steric-hindrance chiral amines. These mutants have been proven to be well suited for industrial production of large-steric-hindrance chiral amines.

The following provides a further detailed description of the present application in conjunction with specific embodiments, which should not be construed as limiting the scope of protection claimed by the present application.

The changes in enzyme stability are represented by the conversion rates at different methanol concentrations and temperatures in Examples 1-4.

### Example 1

On the basis of the parent SEQ ID NO: 1, two rounds of saturation mutagenesis were performed. The specific mutation sites are shown in the table below, and the catalytic activity of the mutants was tested according to the following reaction conditions.

1 mL reaction system included 30 mg of substrate 1 or substrate 2, 300 µL of methanol, 1 mg of PLP, 2 mg of isopropylamine hydrochloride, 300 µL of crude enzyme solution (prepared from 30 mg wet bacterial sludge), pH 8.0, 100 mM phosphate buffer, and reaction at 40°C for 18 hours.

The test results are shown in the table below.

**Table 1:**

| Mutation sites | Conversion rate% | |
|---|---|---|
| | Substrate 1 | Substrate 2 |
| SEQ ID NO: 1 (parent) | + | + |
| V315T | +++ | +++ |
| V315R | ++ | ++ |
| V315D | ++ | ++ |
| V315A | ++ | ++ |
| V315K | ++ | ++ |
| V315H | ++ | ++ |
| V315T+I91Q | ++++ | ++++ |
| V315T+I91M | ++++ | ++++ |
| V315T+I91N | +++++ | ++++ |
| V315T+I91G | ++++ | +++++ |
| V315T+I91F | ++++ | ++++ |
| V315T+I91D | ++++ | ++++ |
| V315T+V124C | +++++ | +++++ |
| V315T+V124S | ++++ | ++++ |
| V315T+V124Y | ++++ | ++++ |
| V315T+V124P | +++++ | ++++ |
| V315T+V124M | ++++ | ++++ |
| V315T+V124A | ++++ | +++++ |

| | | |
|---|---|---|
| Note: in the above table, + represents conversion rate less than 10%, and ++ represents conversion rate greater than or equal to 10% and less than or equal to 20%. +++ represents conversion rate greater than or equal to 20% and less than or equal to 30%, ++++ represents conversion rate greater than or equal to 30% and less than or equal to 40%, +++++ represents conversion rate greater than or equal to 40% and less than or equal to 50%, and ++++++ represents conversion rate greater than or equal to 50% and less than or equal to 60%. | | |

### Example 2

On the basis of Example 1, saturation mutation and combination mutation were continued, and activity screening was performed on the mutations according to the following reaction conditions,
1 mL reaction system included 30 mg of substrate 1 or substrate 2, 400 µL of methanol, 1 mg of PLP, 2 mg of isopropylamine hydrochloride, 300 µL of crude enzyme solution (prepared from 30 mg wet bacterial sludge), pH 8.0, 100 mM phosphate buffer, and reaction at 40°C for 18 hours.

The results are shown in the table below.

**Table 2:**

| Mutation sites | Conversion rate% | |
|---|---|---|
| | Substrate 1 | Substrate 2 |
| V315T+V124C | ++ | ++ |
| V315T+V124C+I91N | ++++ | ++++ |
| V315T+V124C+I91Q | +++ | +++ |
| V315T+V124C+I91M | ++++ | +++ |
| V315T+V124C+I91G | ++++ | ++++ |
| V315T+V124C+I91F | +++ | +++ |
| V315T+I91Q+V124S | ++++ | ++++ |
| V315T+I91Q+V124I | ++++ | +++ |
| V315T+I91Q+V124P | +++ | ++++ |
| V315T+I91Q+V124M | ++++ | +++ |
| V315T+I91Q+V124A | ++++ | +++ |
| V315T+I91Q+V124Y | ++++ | +++ |
| V315T+I91Q+V124S+Y116F | +++++ | +++++ |
| V315T+I91Q+V124S+Y116Y | ++++ | ++++ |
| V315T+I91Q+V124S+Y116A | ++++ | ++++ |
| V315T+I91Q+V124S+Y116R | ++++ | ++++ |
| V315T+V124C+I91N+Y116F | +++++ | +++++ |
| V315T+V124C+I91N+Y116A | ++++ | +++++ |
| V315T+V124C+I91N+Y116S | +++++ | ++++ |
| V315T+V124C+I91N+Y116G | ++++ | +++++ |
| V315T+V124C+I91N+Y116H | +++++ | ++++ |
| V315T+V124C+I91N+Y116L | ++++ | ++++ |
| V315T+V124C+I91N+Y116F+A286I | ++++++ | ++++++ |
| V315T+V124C+I91N+Y116F+A286R | +++++ | +++++ |
| V315T+V124C+I91N+Y116F+A286Q | +++++ | +++++ |
| V315T+V124C+I91N+Y116F+A286F | ++++ | +++++ |
| V315T+V124C+I91N+Y116F+A286D | +++++ | ++++ |
| V315T+V124C+I91N+Y116F+A286M | +++++ | +++++ |

| | | |
|---|---|---|
| Note: in the above table, + represents conversion rate less than 10%, and ++ represents conversion rate greater than or equal to 10% and less than or equal to 20%. +++ represents conversion rate greater than or equal to 20% and less than or equal to 30%, ++++ represents conversion rate greater than or equal to 30% and less than or equal to 40%, +++++ represents conversion rate greater than or equal to 40% and less than or equal to 50%, and ++++++ represents conversion rate greater than or equal to 50% and less than or equal to 60%. | | |

### Example 3

On the basis of Example 2, multiple rounds of saturation mutagenesis were performed, and the catalytic activity of the mutants was tested according to the following reaction conditions:
1 mL reaction system included 30 mg of substrate 1 or substrate 2, 500 µL of methanol, 1 mg of PLP, 2 mg of isopropylamine hydrochloride, 150 µL of crude enzyme solution (prepared from 15 mg wet bacterial sludge), pH 8.0, 100 mM phosphate buffer, and reaction at 40°C for 18 hours.

The results are shown in the table below.

**Table 3:**

| Mutation sites | Conversion rate% | |
|---|---|---|
| | Substrate 1 | Substrate 2 |
| V315T+V124C+I91N+Y116F+A286I | ++ | ++ |
| V315T+V124C+I91N+Y116F+A286I+C418L | +++ | ++++ |
| V315T+V124C+I91N+Y116F+A286I+C418W | +++ | +++ |
| V315T+V124C+I91N+Y116F+A286I+C418R | ++ | +++ |
| V315T+V124C+I91N+Y116F+A286I+C418D | +++ | ++ |
| V315T+V124C+I91N+Y116F+A286I+C418F | +++ | +++ |
| V315T+V124C+I91N+Y116F+A286I+C418A | +++ | ++ |
| V315T+V124C+I91N+Y116F+A286I+T87A | ++++ | ++++ |
| V315T+V124C+I91N+Y116F+A286I+T87Y | ++++ | ++++ |
| V315T+V124C+I91N+Y116F+A286I+T87N | ++++ | +++ |
| V315T+V124C+I91N+Y116F+A286I+T87V | ++++ | ++++ |
| V315T+V124C+I91N+Y116F+A286I+T87F | ++++ | +++ |
| V315T+V124C+I91N+Y116F+A286I+T87E | +++ | ++++ |
| V315T+V124C+I91N+Y116F+A286I+T87A+S301A | +++++ | +++++ |
| V315T+V124C+I91N+Y116F+A286I+T87A+S301G | +++++ | ++++ |
| V315T+V124C+I91N+Y116F+A286I+T87A+S301H | +++++ | ++++ |
| V315T+V124C+I91N+Y116F+A286I+T87A+S301K | +++++ | ++++ |
| V315T+V124C+I91N+Y116F+A286I+T87A+S301W | ++++ | +++++ |
| V315T+V124C+I91N+Y116F+A286I+T87A+S27A | ++++++ | ++++++ |
| V315T+V124C+I91N+Y116F+A286I+T87A+S27V | +++++ | +++++ |
| V315T+V124C+I91N+Y116F+A286I+T87A+S27N | +++++ | +++++ |
| V315T+V124C+I91N+Y116F+A286I+T87A+S27M | +++++ | ++++ |
| V315T+V124C+I91N+Y116F+A286I+T87A+S27E | ++++++ | ++++++ |
| V315T+V124C+I91N+Y116F+A286I+T87A+S27R | +++++ | +++++ |

| | | |
|---|---|---|
| Note: in the above table, + represents conversion rate less than 10%, and ++ represents conversion rate greater than or equal to 10% and less than or equal to 20%. +++ represents conversion rate greater than or equal to 20% and less than or equal to 30%, ++++ represents conversion rate greater than or equal to 30% and less than or equal to 40%, +++++ represents conversion rate greater than or equal to 40% and less than or equal to 50%, and ++++++ represents conversion rate greater than or equal to 50% and less than or equal to 60%. | | |

### Example 4

On the basis of Example 3, multiple rounds of saturation mutagenesis were continued to be performed, and the catalytic activity of the mutants was tested according to the following reaction conditions:
1 mL reaction system included 50 mg of substrate 1 or substrate 2, 600 µL of methanol, 1 mg of PLP, 2 mg of isopropylamine hydrochloride, 100 µL of crude enzyme solution (prepared from 10 mg wet bacterial sludge), pH 8.0, 100 mM phosphate buffer, and reaction at 50°C for 18 hours.

**Table 4:**

| Mutation sites | Conversion rate% | |
|---|---|---|
| | Substrat e 1 | Substrat e 2 |
| V315T+V124C+I91N+Y116F+A286I+T87A+S27A | + | + |
| V315T+V124C+I91N+Y116F+A286I+T87A+S27A+S101A | +++ | +++ |
| V315T+V124C+I91N+Y116F+A286I+T87A+S27A+S101R | +++ | ++ |
| V315T+V124C+I91N+Y116F+A286I+T87A+S27A+S101Y | +++ | ++ |
| V315T+V124C+I91N+Y116F+A286I+T87A+S27A+S101H | +++ | ++ |
| V315T+V124C+I91N+Y116F+A286I+T87A+S27A+R34H | ++ | + |
| V315T+V124C+I91N+Y116F+A286I+T87A+S27A+R34K | ++ | ++ |
| V315T+V124C+I91N+Y116F+A286I+T87A+S27A+R34N | ++ | ++ |
| V315T+V124C+I91N+Y116F+A286I+T87A+S27A+R34T | ++ | +++ |
| V315T+V124C+I91N+Y116F+A286I+T87A+S27A+R34F | ++ | ++ |
| V315T+V124C+I91N+Y116F+A286I+T87A+S27A+T117V | ++ | +++ |
| V315T+V124C+I91N+Y116F+A286I+T87A+S27A+T117M | ++ | ++ |
| V315T+V124C+I91N+Y116F+A286I+T87A+S27A+S301K | ++ | +++ |
| V315T+V124C+I91N+Y116F+A286I+T87A+S27A+S301I | ++ | ++ |
| V315T+V124C+I91N+Y116F+A286I+T87A+S27A+S301G | +++ | ++ |
| V315T+V124C+I91N+Y116F+A286I+T87A+S27A+S301W | ++ | ++ |
| V315T+V124C+I91N+Y116F+A286I+T87A+S27A+S101A+R34H | ++++ | ++++ |
| V315T+V124C+I91N+Y116F+A286I+T87A+S27A+S101A+R34T | ++ | +++ |
| V315T+V124C+I91N+Y116F+A286I+T87A+S27A+S101A+R34N | +++ | +++ |
| V315T+V124C+I91N+Y116F+A286I+T87A+S27A+S101A+R34F | ++++ | +++ |
| V315T+V124C+I91N+Y116F+A286I+T87A+S27A+S101A+R34M | +++ | +++ |
| V315T+V124C+I91N+Y116F+A286I+T87A+S27A+S101A+S301M | +++ | +++ |
| V315T+V124C+I91N+Y116F+A286I+T87A+S27A+S101A+S301K | +++ | +++ |
| V315T+V124C+I91N+Y116F+A286I+T87A+S27A+S101A+S301I | +++ | +++ |
| V315T+V124C+I91N+Y116F+A286I+T87A+S27A+S101A+S301N | ++++ | +++ |
| V315T+V124C+I91N+Y116F+A286I+T87A+S27A+S101A+S301A | +++ | ++++ |
| V315T+V124C+I91N+Y116F+A286I+T87A+S27A+S101A+R34H+T117V | +++++ | +++++ |
| V315T+V124C+I91N+Y116F+A286I+T87A+S27A+S101A+R34H+T117L | +++ | ++++ |
| V315T+V124C+I91N+Y116F+A286I+T87A+S27A+S101A+R34H+T117T | ++++ | ++++ |
| V315T+V124C+I91N+Y116F+A286I+T87A+S27A+S101A+R34H+T117R | ++++ | +++++ |
| V315T+V124C+I91N+Y116F+A286I+T87A+S27A+S101A+R34H+T117S | +++++ | ++++ |
| V315T+V124C+I91N+Y116F+A286I+T87A+S27A+S101A+R34H+T117V+ T285M | ++++++ | ++++++ |
| V315T+V124C+I91N+Y116F+A286I+T87A+S27A+S101A+R34H+T117V+ T285Y | +++++ | ++++++ |
| V315T+V124C+I91N+Y116F+A286I+T87A+S27A+S101A+R34H+T117V+ T285I | ++++++ | +++++ |
| V315T+V124C+I91N+Y116F+A286I+T87A+S27A+S101A+R34H+T117V+ T285A | +++++ | ++++++ |
| V315T+V124C+I91N+Y116F+A286I+T87A+S27A+S101A+R34H+T117V+ T285G | ++++++ | ++++++ |
| V315T+V124C+I91N+Y116F+A286I+T87A+S27A+S101A+R34H+T117V+ T107S | ++++++ | ++++++ |
| V315T+V124C+I91N+Y116F+A286I+T87A+S27A+S101A+R34H+T117V+ T107Q | ++++++ | +++++ |
| V315T+V124C+I91N+Y116F+A286I+T87A+S27A+S101A+R34H+T117V+ T107A | ++++++ | +++++ |
| V315T+V124C+I91N+Y116F+A286I+T87A+S27A+S101A+R34H+T117V+ T107G | ++++++ | ++++++ |

| | | |
|---|---|---|
| Note: in the above table, + represents conversion rate less than 10%, and ++ represents conversion rate greater than or equal to 10% and less than or equal to 20%. +++ represents conversion rate greater than or equal to 20% and less than or equal to 30%, ++++ represents conversion rate greater than or equal to 30% and less than or equal to 40%, +++++ represents conversion rate greater than or equal to 40% and less than or equal to 50%, and ++++++ represents conversion rate greater than or equal to 50% and less than or equal to 60%. | | |

### Example 5

Some of the mutants obtained in the above-mentioned embodiments were selected to test the stability of the enzyme after treatment with high temperature and different organic solvents. The reaction was performed under the following conditions:
1 mL reaction system included 50 mg of substrate 1 or substrate 2, 1 mg of PLP, and 2 mg of isopropylamine hydrochloride. After treatment at 70°C for 1 hour or treatment with methanol, ethanol, acetonitrile, ethyl acetate, dichloromethane, methyl tertiary ether, and n-hexane for 1 hour, 100 µL of crude enzyme solution (prepared from 10 mg wet bacterial sludge) was prepared. The control was the same amount of enzyme solution without any treatment, pH 8.0, 100 mM phosphate buffer, and reaction at 40°C for 18 hours.

The results - are shown in the table below.

**Table 5-1:**

| Mutation sites | Enzym e activity | Relative residual activity (substrate 1) | | | |
|---|---|---|---|---|---|
| | Control | 70°C | Methano I | Ethano I | Acetonitril e |
| SEQ ID NO: 1 (parent) | - | ++ | + | + | + |
| V315T+VI24C+I91N | * | +++ | ++ | ++ | ++ |
| V315T+VI24C+I91N+Y116F | * | +++ | ++ | ++ | ++ |
| V315T+VI24C+I91N+Y116F+A286I | ** | +++ | +++ | +++ | +++ |
| V315T+V124C+I91N+Y116F+A286I+T87A +S27A+S101A | *** | +++ | +++ | +++ | +++ |
| V315T+V124C+I91N+Y116F+A286I+T87A + S27A+S101A+R34H | *** | +++ | +++ | +++ | +++ |
| V315T+V124C+I91N+Y116F+A286I+T87A + S27A+S101A+R34H+T117V | *** | +++ + | +++ | ++++ | +++ |
| V315T+V124C+I91N+Y116F+A286I+T87A + S27A+S101A+R34H+T117V+T285M | **** | +++ + | ++++ | ++++ | ++++ |
| V315T+V124C+I91N+Y116F+A286I+T87A + S27A+S101A+R34H+T117V+T285A | *** | +++ + | ++++ | ++++ | ++++ |

**Table 5-2:**

| Mutation sites | Relative residual activity (substrate 1) | | | |
|---|---|---|---|---|
| | Ethyl acetate | Dichloromethane | Methyl tertiary ether | Heptane |
| SEQ ID NO: 1 (parent) | + | + | + | + |
| V315T+V124C+I91N | ++ | ++ | ++ | ++ |
| V315T+V124C+I91N+Y116F | ++ | ++ | ++ | ++ |
| V315T+V124C+I91N+Y116F+A286I | +++ | ++ | ++ | ++ |
| V315T+V124C+I91N+Y116F+A286I+T87A +S27A+S101A | +++ | +++ | +++ | ++ |
| V315T+V124C+I91N+Y116F+A286I+T87A+ S27A+S101A+R34H | +++ | +++ | +++ | +++ |
| V315T+V124C+I91N+Y116F+A286I+T87A+ S27A+S101A+R34H+T117V | +++ | +++ | ++++ | +++ |
| V315T+V124C+I91N+Y116F+A286I+T87A+ S27A+S101A+R34H+T117V+T285M | ++++ | +++ | ++++ | +++ |
| V315T+V124C+I91N+Y116F+A286I+T87A+ S27A+S101A+R34H+T117V+T285A | ++++ | +++ | ++++ | +++ |

**Table 6-1:**

| Mutation sites | Enzym e activity | Relative residual activity (substrate 2) | | | |
|---|---|---|---|---|---|
| | Control | 70°C | Methano I | Ethano I | Acetonitril e |
| SEQ ID NO: 1 (parent) | - | ++ | + | + | + |
| V315T+V124C+I91N | * | ++ | + | + | ++ |
| V315T+V124C+I91N+Y116F | * | ++ | ++ | ++ | ++ |
| V315T+V124C+I91N+Y116F+A286I | ** | ++ | +++ | +++ | +++ |
| V315T+V124C+I91N+Y116F+A286I+T87A +S27A+S101A | ** | +++ | +++ | +++ | +++ |
| V315T+VI24C+I91N+Y116F+A286I+T87A + S27A+S101A+R34H | *** | +++ | +++ | +++ | +++ |
| V315T+VI24C+I91N+Y116F+A286I+T87A + S27A+S101A+R34H+T117V | *** | +++ + | +++ | +++ | +++ |
| V315T+VI24C+I91N+Y116F+A286I+T87A + S27A+S101A+R34H+T117V+T285M | **** | +++ + | ++++ | ++++ | ++++ |
| V315T+VI24C+I91N+Y116F+A286I+T87A + S27A+S101A+R34H+T117V+T285A | *** | +++ | +++ | ++++ | ++++ |

**Table 6-2:**

| Mutation sites | Relative residual activity (substrate 2) | | | |
|---|---|---|---|---|
| | Ethyl acetate | Dichloromethane | Methyl tertiary ether | Heptane |
| SEQ ID NO: 1 (parent) | + | + | + | + |
| V315T+V124C+I91N | ++ | + | + | + |
| V315T+V124C+I91N+Y116F | ++ | ++ | ++ | ++ |
| V315T+V124C+I91N+Y116F+A286I | +++ | ++ | ++ | ++ |
| V315T+V124C+I91N+Y116F+A286I+T87A +S27A+S101A | ++ | ++ | +++ | ++ |
| V315T+V124C+I91N+Y116F+A286I+T87A+ S27A+S101A+R34H | +++ | +++ | +++ | +++ |
| V315T+V124C+I91N+Y116F+A286I+T87A+ S27A+S101A+R34H+T117V | +++ | +++ | ++++ | +++ |
| V315T+V124C+I91N+Y116F+A286I+T87A+ S27A+S101A+R34H+T117V+T285M | ++++ | ++++ | ++++ | +++ |
| V315T+V124C+I91N+Y116F+A286I+T87A+ S27A+S101A+R34H+T117V+T285A | ++++ | +++ | ++++ | ++++ |

| | | | | |
|---|---|---|---|---|
| Note: 1) in the above-mentioned table, relative residual activity refers to the percentage of enzyme activity measured by enzyme solution properly treated under extreme conditions such as high temperature and organic solvents, compared to the enzyme activity under optimal conditions of enzyme solution without extreme environmental treatment. Under the same treatment conditions, the relative residual activity is high, indicating that the enzyme is more stable under these conditions. 2) In the control, * represents an increase of 0.1-0.5 times in activity compared to the parent; ** represents an increase of 0.5-1.0 times in activity compared to the parent; *** represents an increase of 1.0-1.5 times in activity compared to the parent; and **** represents an increase of 1.5-2 times in activity compared to the parent. 3) + represents relative residual activity greater than or equal to 10% and less than 30%, ++ represents relative residual activity greater than or equal to 30% and less than 50%, +++ represents relative residual activity greater than or equal to 50% and less than 70%, and ++++ represents relative residual activity greater than or equal to 70% and less than 90%. | | | | |

From the above-mentioned embodiment description, it can be seen that:
1) the stability of the mutant obtained in the present application has been significantly improved at high temperatures;
2) in terms of tolerance to organic solvents, in addition to a significant improvement in tolerance to methanol, the tolerance to the six commonly used organic solvents mentioned above has been improved to varying degrees; and
3) in addition, from the above control experiments, it can be seen that enzyme evolution not only improves the stability of mutants, but also enhances enzyme activity to varying degrees.

### Example 6

20 mL of 100 mmol/L phosphate buffer solution, 20 mL of 6 mol/L isopropylamine hydrochloride solution (2 vol), and 60 mL of methanol were added to a 250 mL four-necked bottle at room temperature, and the pH was adjusted to 8.5-9.0. 0.1 g of pyridoxal phosphate and 5 g of 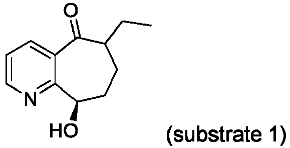 were added, stirred well, and then 2 mL of enzyme solution (0.5 g wet bacterial sludge/mL enzyme solution) of the mutated transaminase mutant (V315T+V124C+I91N+Y116F+A286I+T87A+S27A+S101A+R34H+T117V+T285M) based on SEQ ID NO: 1 was added to adjust the pH to be 8.5-9.0, heated up to 50°C and stirred to react. After the reaction was complete, the system was adjusted to pH 2-3 to denature the protein. After filtration, the filtrate was extracted with 50 mL of methyl tert butyl ether. The pH of the aqueous phase was adjusted to be 12, and the aqueous phase was extracted twice with 50 mL of methyl tert butyl ether. The organic phase was combined, dried with anhydrous magnesium sulfate, and concentrated to no distillate at T<40°C and P≤-0.06 Mpa. The target product 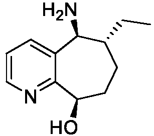 was obtained.

According to HPLC detection, the purity is greater than 99%, the de value is greater than 99%, and the yield is 85%.

### Example 7

20 mL of 100 mmol/L phosphate buffer solution, 20 mL of 6 mol/L isopropylamine hydrochloride solution (2 vol), and 60 mL of methanol were added to a 250 mL four-necked bottle at room temperature, and the pH was adjusted to 8.5-9.0. 0.1 g of pyridoxal phosphate and 5 g of 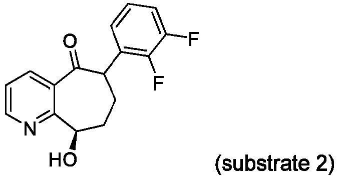 were added, stirred well, and then 2 mL of enzyme solution (0.5 g wet bacterial sludge/mL enzyme solution) of the mutated transaminase mutant (V315T+V124C+I91N+Y116F+A286I+T87A+S27A+S101A+R34H+T117V+T285M) based on SEQ ID NO: 1 was added to adjust the pH to be 8.5-9.0, heated up to 50°C and stirred to react. After the reaction was complete, the system was adjusted to pH 2-3 to denature the protein. After filtration, the filtrate was extracted with 50 mL of methyl tert butyl ether. The pH of the aqueous phase was adjusted to be 12, and the aqueous phase was extracted twice with 50 mL of methyl tert butyl ether. The organic phase was combined, dried with anhydrous magnesium sulfate, and concentrated to no distillate at T<40°C and P≤-0.06 Mpa. The target product 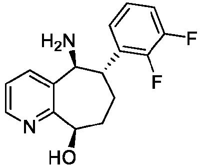 was obtained.

According to HPLC detection, the purity is greater than 99%, the de value is greater than 99%, and the yield is 84%.

### Example 8

20 mL of 100 mmol/L phosphate buffer solution, 20 mL of 6 mol/L isopropylamine hydrochloride solution (16 vol), and 60 mL of methanol were added to a 250 mL four-necked bottle at room temperature, and the pH was adjusted to 8.5-9.0. 0.1 g of pyridoxal phosphate and 5 g of 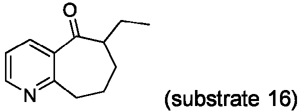 were added, stirred well, and then 2 mL of enzyme solution (0.5 g wet bacterial sludge/mL enzyme solution) of the mutated transaminase mutant (V315T+V124C+I91N+Y116F+A286I+T87A+S27A+S101A+R34H+T117V+T285M) based on SEQ ID NO: 1 was added to adjust the pH to be 8.5-9.0, heated up to 50°C and stirred to react. After the reaction was complete, the system was adjusted to pH 2-3 to denature the protein. After filtration, the filtrate was extracted with 50 mL of methyl tert butyl ether. The pH of the aqueous phase was adjusted to be 12, and the aqueous phase was extracted twice with 50 mL of methyl tert butyl ether. The organic phase was combined, dried with anhydrous magnesium sulfate, and concentrated to no distillate at T<40°C and P≤-0.06 Mpa. The target product 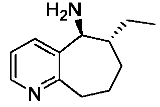 was obtained.

According to HPLC detection, the purity is greater than 99%, the de value is greater than 99%, and the yield is 75%.

### Example 9

20 mL of 100 mmol/L phosphate buffer solution, 20 mL of 6 mol/L isopropylamine hydrochloride solution (17 vol), and 60 mL of methanol were added to a 250 mL four-necked bottle at room temperature, and the pH was adjusted to 8.5-9.0. 0.1 g of pyridoxal phosphate and 5 g of 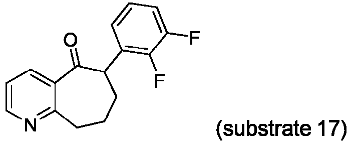 were added, stirred well, and then 2 mL of enzyme solution (0.5 g wet bacterial sludge/mL enzyme solution) of the mutated transaminase mutant (V315T+V124C+I91N+Y116F+A286I+T87A+S27A+S101A+R34H+T117V+T285M) based on SEQ ID NO: 1 was added to adjust the pH to be 8.5-9.0, heated up to 50°C and stirred to react. After the reaction was complete, the system was adjusted to pH 2-3 to denature the protein. After filtration, the filtrate was extracted with 50 mL of methyl tert butyl ether. The pH of the aqueous phase was adjusted to be 12, and the aqueous phase was extracted twice with 50 mL of methyl tert butyl ether. The organic phase was combined, dried with anhydrous magnesium sulfate, and concentrated to no distillate at T<40°C and P≤-0.06 Mpa. The target product 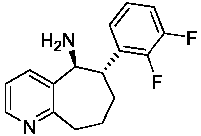 was obtained.

According to HPLC detection, the purity is greater than 99%, the de value is greater than 99%, and the yield is 80%.

### Example 10

The enzyme solution of the transaminase mutant (V315T+V124C+I91N+Y116F+A286I+T87A+S27A+S101A+R34H+T117V+T285M) mutated based on SEQ ID NO: 1 was used to perform catalytic reaction to substrates 3-15, following the catalytic synthesis steps of Examples 6-9. The results are shown in the following table:

**Table 7:**

| Substrate | Product purity | De value | Yield |
|---|---|---|---|
| 3 | >99% | >99% | 81% |
| 4 | >99% | >98% | 78% |
| 5 | >99% | >99% | 82% |
| 6 | >98% | >98% | 83% |
| 7 | >98% | >99% | 82% |
| 8 | >99% | >99% | 80% |
| 9 | >98% | >99% | 81% |
| 10 | >99% | >99% | 84% |
| 11 | >99% | >99% | 82% |
| 12 | >99% | >99% | 85% |
| 13 | >98% | >99% | 87% |
| 14 | >99% | >99% | 78% |
| 15 | >99% | >99% | 83% |

From the above description, it can be seen that the above-mentioned embodiments of the present disclosure achieve the following technical effects:
1) by evolving transaminase, mutants with improved activity and stability have been obtained, which can be used for the synthesis of various large-steric-hindrance chiral amines under high temperature and high organic solvent conditions, without the need for heavy metal catalysts and toxic reagents, achieving green chemistry.
2) This transaminase mutant has high biocatalytic activity, enzyme stability, high reaction substrate concentration, high product yield, greatly reduces three wastes, and saves production costs.

The embodiments described above are just preferred embodiments of the present disclosure and are not used for limiting the present disclosure. For those skilled in the art, the present disclosure may have various modifications and variations. Any modification, equivalent replacement, improvement and the like made within the spirit and principle of the present disclosure shall be still included in the protection scope of the present disclosure.

## Claims

1. A transaminase mutant, comprising:
(a) a protein having the amino acid sequence of SEQ ID NO: 1; or
(b) a protein having an amino acid mutation at one or more of the following sites in (a): V315, I91, V124, Y116, A286, C418, T87, S301, S27, V31, R34, M64, A74, R77, S101, T117, N151, L213, T285, T107, or L449, and having the function of transaminase; and
(c) a protein having more than 80% identity with the amino acid sequence defined in any one of (a) and (b) and having the function of transaminase.

2. The transaminase mutant according to claim 1, wherein the amino acid mutation of (b) is each independently selected from the following:
V315T, V315R, V315D, V315A, V315K or V315H;
I91Q, I91M, I91N, I91G, I91F or I91D;
V124C, V124S, V124Y, V124P, V124M, V124A or V124I;
Y116F, Y116Y, Y116A, Y116R, Y116S, Y116G, Y116H or Y116L;
A286I, A286R, A286Q, A286F, A286D, A286M or A2861;
C418L, C418W, C418R, C418D, C418F or C418A;
T87A, T87Y, T87N, T87V, T87F or T87E;
S301A, S301G, S301H, S301K, S301W, S301I or S301N;
S27A, S27V, S27N, S27M, S27E, S27R or S27;
S101A, S101R, S101Y or S101H;
R34H, R34K, R34N, R34T, R34F or R34M;
T117V, T117M, T117L, T117T, T117R or T117S;
T285M, T285Y, T285I, T285A or T285G;
T107S, T107Q, T107A or T107G;
wherein the letter before a number represents the original amino acid, and a letter after the number represents a mutated amino acid;
preferably, the protein in the (c) is a protein having more than 85%, preferably more than 90%, more preferably more than 95%, and further more preferably more than 99% identity with the amino acid sequence defined in any of (a) and (b) and having the function of transaminase.

3. The transaminase mutant according to claim 2, wherein the transaminase mutant comprise any one of the following amino acid mutations:
V315T;
V315R;
V315D;
V315A;
V315K;
V315H;
V315T+I91Q;
V315T+I91M;
V315T+I91N;
V315T+I91G;
V315T+I91F;
V315T+I91D;
V315T+V124C;
V315T+V124S;
V315T+V124Y;
V315T+V124P;
V315T+V124M;
V315T+V124A;
V315T+V124C+I91N;
V315T+V124C+I91Q;
V315T+V124C+I91M;
V315T+V124C+I91G;
V315T+V124C+I91F;
V315T+I91Q+V124S;
V315T+I91Q+V124I;
V315T+I91Q+V124P;
V315T+I91Q+V124M;
V315T+I91Q+V124A;
V315T+I91Q+V124Y;
V315T+I91Q+V124S+Y116F;
V315T+I91Q+V124S+Y116Y;
V315T+I91Q+V124S+Y116A;
V315T+I91Q+V124S+Y116R;
V315T+V124C+I91N+Y116F;
V315T+V124C+I91N+Y116A;
V315T+V124C+I91N+Y116S;
V315T+V124C+I91N+Y116G;
V315T+V124C+I91N+Y116H;
V315T+V124C+I91N+Y116L;
V315T+V124C+I91N+Y116F+A286I,
V315T+V124C+I91N+Y116F+A286R;
V315T+V124C+I91N+Y116F+A286Q;
V315T+V124C+I91N+Y116F+A286F;
V315T+V124C+I91N+Y116F+A286D;
V315T+V124C+I91N+Y116F+A286M;
V315T+V124C+I91N+Y116F+A286I+C418L;
V315T+V124C+I91N+Y116F+A286I+C418W;
V315T+V124C+I91N+Y116F+A286I+C418R;
V315T+V124C+I91N+Y116F+A286I+C418D;
V315T+V124C+I91N+Y116F+A286I+C418F;
V315T+V124C+I91N+Y116F+A286I+C418A;
V315T+V124C+I91N+Y116F+A286I+T87A;
V315T+V124C+I91N+Y116F+A286I+T87Y;
V315T+V124C+I91N+Y116F+A286I+T87N;
V315T+V124C+I91N+Y116F+A286I+T87V;
V315T+V124C+I91N+Y116F+A286I+T87F;
V315T+V124C+I91N+Y116F+A286I+T87E;
V315T+V124C+I91N+Y116F+A286I+T87A+S301A;
V315T+V124C+I91N+Y116F+A286I+T87A+S301G;
V315T+V124C+I91N+Y116F+A286I+T87A+S301H;
V315T+V124C+I91N+Y116F+A286I+T87A+S301K;
V315T+V124C+I91N+Y116F+A286I+T87A+S301W;
V315T+V124C+I91N+Y116F+A286I+T87A+S27A;
V315T+V124C+I91N+Y116F+A286I+T87A+S27V;
V315T+V124C+I91N+Y116F+A286I+T87A+S27N;
V315T+V124C+I91N+Y116F+A286I+T87A+S27M;
V315T+V124C+I91N+Y116F+A286I+T87A+S27E;
V315T+V124C+I91N+Y116F+A286I+T87A+S27R;
V315T+V124C+I91N+Y116F+A286I+T87A+S27A+S101A;
V315T+V124C+I91N+Y116F+A286I+T87A+S27A+S101R;
V315T+V124C+I91N+Y116F+A286I+T87A+S27A+S101Y;
V315T+V124C+I91N+Y116F+A286I+T87A+S27A+S101H;
V315T+V124C+I91N+Y116F+A286I+T87A+S27A+R34H;
V315T+V124C+I91N+Y116F+A286I+T87A+S27A+R34K;
V315T+V124C+I91N+Y116F+A286I+T87A+S27A+R34N;
V315T+V124C+I91N+Y116F+A286I+T87A+S27A+R34T;
V315T+V124C+I91N+Y116F+A286I+T87A+S27A+R34F;
V315T+V124C+I91N+Y116F+A286I+T87A+S27A+T117V;
V315T+V124C+I91N+Y116F+A286I+T87A+S27A+T117M;
V315T+V124C+I91N+Y116F+A286I+T87A+S27A+S301K;
V315T+VI24C+I91N+Y116F+A286I+T87A+S27A+S301I;
V315T+V124C+I91N+Y116F+A286I+T87A+S27A+S301G;
V315T+V124C+I91N+Y116F+A286I+T87A+S27A+S301W;
V315T+V124C+I91N+Y116F+A286I+T87A+S27A+S101A+R34H;
V315T+V124C+I91N+Y116F+A286I+T87A+S27A+S101A+R34T;
V315T+V124C+I91N+Y116F+A286I+T87A+S27A+S101A+R34N;
V315T+V124C+I91N+Y116F+A286I+T87A+S27A+S101A+R34F;
V315T+V124C+I91N+Y116F+A286I+T87A+S27A+S101A+R34M;
V315T+V124C+I91N+Y116F+A286I+T87A+S27A+S101A+S301M;
V315T+V124C+I91N+Y116F+A286I+T87A+S27A+S101A+S301K;
V315T+V124C+I91N+Y116F+A286I+T87A+S27A+S101A+S301I;
V315T+V124C+I91N+Y116F+A286I+T87A+S27A+S101A+S301N;
V315T+V124C+I91N+Y116F+A286I+T87A+S27A+S101A+S301A;
V315T+V124C+I91N+Y116F+A286I+T87A+S27A+S101A+R34H+T117V;
V315T+V124C+I91N+Y116F+A286I+T87A+S27A+S101A+R34H+T117L;
V315T+V124C+I91N+Y116F+A286I+T87A+S27A+S101A+R34H+T117T;
V315T+V124C+I91N+Y116F+A286I+T87A+S27A+S101A+R34H+T117R;
V315T+V124C+I91N+Y116F+A286I+T87A+S27A+S101A+R34H+T117S;
V315T+V124C+I91N+Y116F+A286I+T87A+S27A+S101A+R34H+T117V+T285M;
V315T+V124C+I91N+Y116F+A286I+T87A+S27A+S101A+R34H+T117V+T285Y;
V315T+V124C+I91N+Y116F+A286I+T87A+S27A+S101A+R34H+T117V+T285I;
V315T+V124C+I91N+Y116F+A286I+T87A+S27A+S101A+R34H+T117V+T285A;
V315T+V124C+I91N+Y116F+A286I+T87A+S27A+S101A+R34H+T117V+T285G;
V315T+V124C+I91N+Y116F+A286I+T87A+S27A+S101A+R34H+T117V+T107S;
V315T+V124C+I91N+Y116F+A286I+T87A+S27A+S101A+R34H+T117V+T107Q;
V315T+V124C+I91N+Y116F+A286I+T87A+S27A+S101A+R34H+T117V+T107A; or
V315T+V124C+I91N+Y116F+A286I+T87A+S27A+S101A+R34H+T117V+T107G.

4. A DNA molecule encoding the transaminase mutant according to any one of claims 1 to 3.

5. A recombinant plasmid connected with the DNA molecule according to claim 4.

6. A host cell transformed with the recombinant plasmid according to claim 5.

7. A method for preparing a chiral amine compound, comprising performing a transamination reaction with the transaminase mutant according to any one of claims 1 to 3 and a ketone-based substrate as shown in a formula I under the action of an amino donor, to obtain the chiral amine compound, 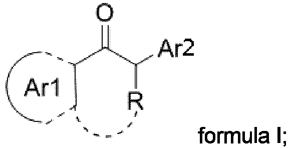
Ar1 is selected from a first substituted aryl group, a first unsubstituted aryl group, a substituted arylene group or an unsubstituted arylene group, a substituted heteroarylene group or an unsubstituted heteroarylene group;
Ar2 is selected from a second substituted aryl group, a second unsubstituted aryl group, a substituted cycloalkyl group or an unsubstituted cycloalkyl group, an alkyl group or an alkylene group;
R is selected from H, an alkyl group, an alkylene group or an alkylidene group, the number of C atoms in the alkyl group, alkylene group or alkylidene group is selected from 1 to 5, and the alkyl group, alkylene group or alkylidene group comprises a substituted alkyl group, alkylene group or alkylidene group, or an unsubstituted alkyl group, alkylene group or alkylidene group;
when R is selected from an alkyl group, alkylene group or alkylidene group, the alkyl group, alkylene group or alkylidene group is linked to the Ar1 and/or the Ar2 to form a ring;
the substituents in the first substituted aryl group, the substituted arylene group, the substituted heteroarylene group, the second substituted aryl group, or the substituted alkyl group, alkylene group or alkylidyne group are each independently selected from a halogen, a hydroxyl group, an amino group, a methyl group, an ethyl group or -CH₂CH₂OH; and
the heteroatoms in the substituted heteroarylene group are selected from N, O or S.

8. The method according to claim 7, wherein the substituents in the first substituted aryl group, the second substituted aryl group or the substituted arylene group are each independently selected from the halogen.

9. The method according to claim 8, wherein the substituent groups are each independently located at any one or more positions of ortho-, meta- or para-position of the first substituted aryl group, the second substituted aryl group or the substituted arylene group.

10. The method according to claim 9, wherein the halogen is selected from F, Cl or Br.

11. The method according to claim 10, wherein,
the Ar1 is selected from the unsubstituted heteroarylene group,
the Ar2 is selected from the second substituted aryl group, and the substituent of the second substituted aryl group is selected from the halogen,
the R is selected from the substituted alkylene group, the number of C atoms in the substituted alkylene group is selected from 1 to 5, and the substituent is selected from a hydroxyl group, and
the substituted alkylene group is linked to the Ar1 to form a ring.

12. The method according to claim 10, wherein,
the Ar1 is selected from the first substituted aryl group or the first unsubstituted aryl group, and the substituent of the first substituted aryl group is selected from a methyl group or Cl,
the Ar2 is selected from the unsubstituted cycloalkyl group, alkyl group or second unsubstituted aryl group, the number of C atoms in the unsubstituted cycloalkyl group is selected from 3 to 5, and the alkyl group is selected from an isopropyl group or an ethyl group,
the R is selected from the alkyl group, and the alkyl group is selected from an isopropyl group, a methyl group, or an ethyl group.

13. The method according to claim 10, wherein,
the Ar1 is selected from the first substituted aryl group or the first unsubstituted aryl group, and the substituent of the first substituted aryl group is selected from a methyl group or -CH₂CH₂OH,
the Ar2 is selected from the second unsubstituted aryl group or alkyl group, the alkyl group is selected from a methyl group or an isopropyl group,
the R is selected from a substituted alkylene group, the number of C atoms in the substituted alkylene group is selected from 1 to 5, and the substituent is selected from a hydroxyl group, and
the substituted alkylene group is linked to the Ar1 to form a ring.

14. The method according to any one of claims 10 to 13, wherein the ketone-based substrate is selected from
